# EUROPÄISCHE PATENTSCHRIFT

(11) **EP 1 359 226 B1**
(45) Veröffentlichungstag und Bekanntmachung des Hinweises auf die Patenterteilung: **22.11.2006**
(21) Anmeldenummer: 03009217.5
(22) Anmeldetag: 23.04.2003
(51) Int. Cl.: C12Q 1/02, G01N 33/58, G01N 33/543

(54) **Verfahren und Vorrichtung zum Nachweis biologisch aktiver Substanzen**
Method and device for detection of biologically active substances
Procédé et dispositif pour la détection des composés bioactifs

(30) Priorität: 29.04.2002 DE 10219031; 05.06.2002 US 386045 P
(43) Veröffentlichungstag der Anmeldung: 05.11.2003
(73) Patentinhaber: Bayer Innovation GmbH, 40225 Düsseldorf (DE)
(72) Erfinder: Kreiss, Wolfgang, Dr., 51467 Bergisch Gladbach (DE); Eberz, Günther, Dr., 51519 Odenthal (DE); Rast, Hans-Georg, Dr., 51519 Odenthal (DE); Weisemann, Claus, Dr., Clayton, NC 27520 (US)
(74) Vertreter: Lütjens, Henning

(56) Entgegenhaltungen:
- US-A- 6 017 722
- US-B1- 6 340 572
- WEINS C ET AL: "Toxicological evaluation of harmful substances by in situ enzymatic and biological detection in high-performance thin-layer chromatography" JOURNAL OF CHROMATOGRAPHY A, ELSEVIER SCIENCE, NL, Bd. 750, Nr. 1-2, 25. Oktober 1996 (1996-10-25), Seiten 403-407, XP004069778 ISSN: 0021-9673
- DATABASE BIOSIS [Online] BIOSCIENCES INFORMATION SERVICE, PHILADELPHIA, PA, US; 1994 KUO ALAN ET AL: "Multiple N-Acyl-L-Homoserine Lactone Autoinducers of Luminescence in the Marine Symbiotic Bacterium Vibrio fischeri" Database accession no. PREV199598083021 XP002259766 & JOURNAL OF BACTERIOLOGY, Bd. 176, Nr. 24, 1994, Seiten 7558-7565, ISSN: 0021-9193
- DATABASE BIOSIS [Online] BIOSCIENCES INFORMATION SERVICE, PHILADELPHIA, PA, US; 2001 LEE BAEK-SEOK ET AL: "Statistical optimization of bioluminescence of Photobacterium phosphoreum KCTC2852" Database accession no. PREV200100473696 XP002259767 & JOURNAL OF BIOSCIENCE AND BIOENGINEERING, Bd. 92, Nr. 1, 2001, Seiten 72-76, ISSN: 1389-1723
- DATABASE BIOSIS [Online] BIOSCIENCES INFORMATION SERVICE, PHILADELPHIA, PA, US; 1980 SHENDEROV A N ET AL: "EFFECT OF AMINO-ACIDS ON THE LUMINESCENT SYSTEM INDUCTION IN PHOTOBACTERIUM-BELOZERSKII" Database accession no. PREV198171048027 XP002259765 & PRIKLADNAYA BIOKHIMIYA I MIKROBIOLOGIYA, Bd. 16, Nr. 2, 1980, Seiten 162-171, ISSN: 0555-1099
- RODICHEVA E.K. ET AL: 'Growth and luminescence of luminous bacteria promoted by agents of microbial origin' JOURNAL OF BIOLUMINESCENCE AND CHEMILUMINESCENCE Bd. 8, Nr. 6, 1993, Seiten 293 - 299
- Casein Peptone E1 composition, http://www.organotechnie.com/Doc.anglais/19 546A.pdf
- Casein Peptone N3 composition, http://www.organotechnie.com/Doc.anglais/19 305A.pdf
- Yeast extract composition: http://www.organotechnie.com/Doc.anglais/19 512A.pdf

## Beschreibung

Lumineszenztests haben für die Untersuchung der biologischen Aktivität von Substanzen allgemeine Bedeutung erhalten und werden z.B. in ökotoxikologischen Anwendungen oder beim Screening von Prüfsubstanzen der pharmazeutischen Forschung eingesetzt. Die Testverfahren beruhen auf der direkten Beeinflussung von Biolumineszenzvorgängen durch Substanzen und zeigen biologische Wirkung bzw. Toxizität durch Änderung der Lumineszenzintensität an. Dabei werden toxische Substanzeigenschaften häufig durch Verringerung der Biolumineszenz nachgewiesen, während spezifische biologische Wirkungen in der Regel durch die Stimulation von Reportergensystemen auf Biolumineszenzbasis selektiv erfasst werden.

Ein verbreitet angewendetes Verfahren zur Bestimmung der Toxizität nutzt die Verringerung der Lumineszenz von Leuchtbakterien in Gegenwart toxischer Substanzen. Nach dieser Methode wird die zu prüfende Substanz zu einer Suspension lumineszenter Bakterien gegeben. Toxische Substanzen reduzieren die Intensität der Lumineszenz und können auf diese Weise erfasst werden [A. A. Bulich, Bioluminescence Assays, S. 57-74 in G. Bitton und B. J. Dutka, Toxicity Testing Using Microorganisms, Vol 1, 1986, CRC Press, Boca Raton]. Für komplexe Substanzgemische, die bei realen Proben häufig vorliegen, liefert dieses Testverfahren lediglich summarische Resultate und lässt keine Rückschlüsse auf die aktive Komponente zu. Darüber hinaus kommt es beim gleichzeitigen Vorliegen von toxischen Substanzen (Lumineszenzverringerung) und Verbindungen, die die Lumineszenz stimulieren zu falschen Bewertungen derartiger Proben.

Zur sicheren Beurteilung der biologischen Aktivität von Gemischen müssen diese in Einzelkomponenten zerlegt und nachfolgend Komponenten-weise geprüft werden. Ein direktes Verfahren hierzu beruht auf der Kopplung von Biolumineszenz mit chromatographischen Trenntechniken (chromatographische Wirkungsdetektion). Dabei werden Substanzgemische dünnschichtchromatographisch getrennt und toxische Substanzen durch Beschichtung der Dünnschichtchromatographie-Platte mit Leuchtbakterien über die Lumineszenzminderung detektiert [US Patent 6,017,722; US Patent 6,340,572; US Patent 6,238,928; G. Eberz, H.-G. Rast, K. Burger, W. Kreiss, C. Weisemann, *Chromatographia* **43,** 5 (1996)].

Die grundsätzlich leistungsfähige chromatographische Wirkungsdetektion nach dem bekannten Stand der Technik hat sich bisher in der Praxis nicht breit durchsetzen können, da die erfolgreiche Anwendung gleichzeitig analysentechnisches und mikrobiologisches Know How und entsprechend umfangreiche Laborausstattungen erfordert.

Zusätzlich zur chromatographischen Substanztrennung muss für den Detektionsschritt eine frische Kultur mit vitalen lumineszenten Bakterien in ausreichender Menge bereitgestellt werden. Die nach dem Stand der Technik ausgeführte Lumineszenzdetektion liefert auf der Dünnschichtchromatographieplatte nur geringe Lumineszenz, so dass die Detektion nur mit hohem Aufwand, z. B. über höchstsensitive Imagingsysteme mit Restlichtverstärkung möglich wird.

Der in US Patent 6,340,572 beschriebene Kit sieht die Anwendung eines Verdünnungsmittels für das biolumineszente Agens vor, d. h. für die Herstellung des Detektionsmittels werden dort zwei Komponenten und ein Verdünnungsschritt konzipiert.

Das in US Patent 6,017,722 beschriebene Verfahren weist die dort beschriebene Einschränkung hinsichtlich der Dauer der Biolumineszenz auf dem Chromatographiemedium auf. Diese Einschränkung folgt aus Verdunstungsprozessen, die die Vitalität der Mikroorganismen beeinträchtigen.

Für *Vibrio harveyi* ist die Lumineszenzsteigerung durch Boratdiester bekannt [X. Chen, S. Schauder, N. Potier, A. van Dorsselaer, I. Pelczer, B.L. Bassler, F.M. Hughson, Nature, Vol. 415, 545, 2002]. Es ist weiterhin bekannt, dass N-Acylhomoserinlactone geeignet sind, die als bakterielle Signalstoffe die Biolumineszenz in *Vibrio fischeri* stimulieren [J. Throup, M.K. Winson, N.J. Bainton, B.W. Bycroft, G.S.A.B. Stewart; Proceedings of the 8^{th} International Symposium of Bioluminescence and Chemiluminescence, Cambridge 1994, S. 89- 92, John Wiley].

Die in US Patent 6,017,722 beschriebene Beschichtung der Platte durch eine Spraytechnik weist in der Praxis zahlreiche Nachteile auf. Mit Labor-üblichen Apparaturen gelingt keine gleichmäßige Beschichtung der Oberfläche. Dies wird durch die in US Patent 6,017,722 angegebenen Beispiele deutlich belegt. Durch Verdunstungsprozesse während des Sprühvorgangs wird ein Teil der Bakterien geschädigt, so dass nur reduzierte Lumineszenzausbeuten erreicht werden. Darüber hinaus ist beim Arbeiten mit Mikroorganismen die Aerosolbildung aus Gründen des Arbeitsschutzes zu vermeiden, d. h. eine Beschichtung durch Sprühen mit lebenden Bakterien erfordert eine wirksame Absaugeinrichtung.

Ch. Weins, H. Jork, "Toxicological evaluation of harmful substances by in-situ enzymatic and biological detection in high-performance thin-layer chromatography", Journal of Chromatography A, 750 (1996), 403-407 beschreibt ein Verfahren zum Nachweis biologisch aktiver Substanzen unter Verwendung von lumineszierenden Mikroorganismen. Eine gezielte Stimulierung der Biolumineszenz durch die Lumineszenz der Mikroorganismen gezielt induzierende Borverbindungen, N-Acylhomoserinlactone oder deren biochemische Vorläufersubstanzen oder Chinolone wird jedoch nicht offenbart.

Aufgabe der Erfindung ist es, ein Verfahren und ein Kit zum Nachweis biologisch aktiver Substanzen bereitzustellen, dass für den praktischen Einsatz der chromatographischen Wirkungsdetektion die Einschränkungen des bisherigen Standes der Technik überwindet und eine für die Detektion ausreichend stabile bzw. langandauernde Lumineszenzemission auch bei geringer Konzentration der Mikroorganismen bereitstellt.

Die Lösung der erfindungsgemäßen Aufgabe besteht in einem Verfahren zur Detektion biologisch aktiver Substanzen enthaltend die Schritte:
a) Bereitstellung eines Test-Trägers mit zu prüfenden Substanzen, insbesondere einer Dünnschichtchromatographie-, Elektrophoreseplatte oder eines Substanzarrays,
b) Bereitstellung einer Suspension mit Mikroorganismen der Species Vibrio fischeri oder einer genetisch modifizierten Variante von V. fischeri ,
c) Beschichtung des Test-Trägers mit der Mikroorganismen-Suspension durch Tauchen in die Mikroorganismen-Suspension,
d) Nachweis der biologisch aktiven Substanzen auf dem Test-Träger durch Detektion der Lumineszenzänderung der Mikroorganismen-Suspension,
e) Stimulation der Lumineszenz der Mikroorganismen vor oder während der Detektion, dadurch gekennzeichnet, dass die Suspension einen Zusatz von Asparaginsäure in einer Konzentration zwischen 100 mg/l und 500 mg/l enthält, sowie dass
f) die Lumineszenz der Mikroorganismen durch Borverbindungen, N-Acylhomoserinlactone oder deren biochemische Vorläufersubstanzen oder Chinolone stimuliert wird.

Das erfindungsgemäße Verfahren zeigt die Lumineszenzsteigerung infolge verbesserten Wachstums unter Zusatz von Asparaginsäure zum Anzuchtmedium.

Es werden dem Anzuchtmedium Asparaginsäure in einer Konzentration zwischen 100 mg/l und 500 mg/l als Zusatz zur Stimulierung des Wachstums und optional Borverbindungen, N-Acylhomoserinlactone oder deren biochemische Vorläufersubstanzen oder Chinolone als Lumineszenz stimulierende Substanzen zugesetzt.

In der Regel zeigen biologische Wirkungen eine ausgeprägte Zeitabhängigkeit. Mit dem erfindungsgemäßen Verfahren können akut toxische Wirkungen von antibiotischen Effekten, die erst nach längerer Einwirkungszeit auftreten, gut unterschieden werden. Zur Untersuchung dieser Zeitabhängigkeit benötigt man hinreichende Stabilität der Lumineszenz auf dem Träger über den Beobachtungszeitraum. Für die Regulierung und Verlängerung, also die zeitliche Stabilisierung der Biolumineszenz, können Substanzen eingesetzt werden, die z.B. ein rasches Austrocknen des mit Mikroorganismen beschichteten Trägers verhindern. Für diesen Zweck eignen sich insbesondere Oligomere und Polymere, insbesondere biokompatible und hydrophile, z.B. auf Polysaccharid-Basis. Bevorzugt werden hierfür Acrylate, Polyvinylpyridine, Polyethylenglykole, Polyetherderivate, Polysaccharide, Dextrane, modifizierte Zellulosen, Peptide und Proteine eingesetzt, besonders bevorzugt in Konzentration bis zu 2 % des sie enthaltenden Mediums.

Die Anzuchtlösung für die Mikroorganismen-Suspension kann die stimulierenden Substanzen für die Lumineszenz der Mikroorganismen oder Bestandteile zur Regulierung und Verlängerung der Lumineszenzdauer der Mikroorganismen enthalten.

Mikroorganismen-Suspensionen können aus gefriergetrockneten Mikroorganismen oder gefrorenen Zellkonzentraten durch Rekonstitution mit einem Rekonstitutionsmedium gewonnen werden. Für die rasche Rekonstitution gefriergetrockneter oder durch Zentrifugation konzentrierter Mikroorganismen ist eine Stimulation der Lumineszenz oder Regulierung und Verlängerung der Lumineszenzdauer der Mikroorganismen durch eine Zugabe entsprechender Substanzen zum Verdünnungsmedium oder Rekonstitutionsmedium besonders vorteilhaft, da ohne Stimulation nur geringe Lumineszenzintensitäten oder -dauern erreicht werden.

Bei der Verdünnung von Mikroorganismen-Suspensionen mit Induktor-haltigen Medien ist es ebenfalls vorteilhaft dem Verdünnungsmedium Substanzen zur Lumineszenzstimulation oder -verlängern zuzugeben. Damit werden geringere Mengen lumineszenter Mikroorganismen benötigt ohne Einbußen bei der Qualität des Nachweises der Lumineszenz hinnehmen zu müssen, so dass sich der Aufwand für die Bereitstellung verringert.

Eine Lumineszenzsteigerung kann weiterhin durch eine hohe Zelldichte von mehr als 2*10⁹ Zellen/ml in der Mikroorganismen-Suspension erreicht werden, zum Beispiel dadurch, dass zum Anzuchtmedium der Mikroorganismen Aminosäuren oder anderen Carbonsäuren, insbesondere Asparaginsäure zugesetzt werden.

Als lumineszente Mikroorganismen werden die marinen Leuchtbakterien *Vibrio fischeri* verwendet.

Der Träger mit den biologisch aktiven Substanzen kann eine Dünnschichtchromatographieplatte oder ein Elektrophoresegel oder ein anderes, bevorzugt planares, Trennsystem sein, auf dem sich die biologisch aktiven Substanzen in Form von Zonen befinden. Die biologisch aktiven Substanzen können sich auch auf einem Träger in Form von Spots eines Substanzarrays befinden.

Die Detektion der Lumineszenz der Mikroorganismen-Suspension kann durch photographische Verfahren oder Imagingtechniken erfolgen.

Die Beschichtung des Trägers kann durch Tauchen des Trägers in eine Suspension von Mikroorganismen erfolgen. Während des Tauchvorgangs findet auf der zu beschichtenden Oberfläche eine Anreicherung der lumineszenten Mikroorganismen statt. So kann z.B. die Suspension von *Vibrio fischeri* aus einer Übernachtkultur bis auf das mehr als fünffache Volumen verdünnt werden und es wird immer noch die sichere Detektion toxischer Substanzen z.B. auf einer mit Kieselgel beschichteten Dünnschichtplatte erreicht. Dieser Effekt ist überraschend, da man beim pH-Wert der Tauchsuspension (ca. pH 7) von einer negativen Ladung der Kieselgelmatrix und gleichzeitig einer negativen Oberflächenladung der Leuchtbakterien ausgehen muss. Eine Erklärungsmöglichkeit für dieses unerwartete Verhalten bietet die Kompensation der elektrostatischen Kräfte durch andere Wechselwirkungen sowie eine reduzierte negative Ladungsdichte für die Bakterienhülle. Messungen des Zetapotentials von *Vibrio fischeri* in der Tauchlösung stützen diese Annahme.

Durch das Tauchen erfolgt außerdem eine raschere Beschichtung des Trägers und es ergibt sich damit die Möglichkeit von zeitabhängigen Messungen bereits nach kurzen Einwirkungszeiten. Auf diese Weise können akute toxische Effekte bereits innerhalb weniger Sekunden nachgewiesen werden. Weiterhin bleibt die Auftrennung von Substanzen auf dem Träger mit hoher Qualität erhalten, da in der kurzen Zeit kaum eine Diffusion der Substanzzonen stattfindet.

Die Beschichtung des Trägers erfolgt bevorzugt homogen, was einen gleichmäßigen Hintergrund und ein verbessertes Signal/Rausch-Verhältnis bei der Lumineszenz-messung zur Folge hat.

Die biologische Aktivität kann, abhängig vom verwendeten biologischen System, sowohl eine Verringerung wie auch eine Zunahme der Lumineszenz bewirken. In einem Messregime kann der zeitliche Verlauf der Inhibierung bzw. Stimulierung der Lumineszenz der Mikroorganismen-Suspension aufgezeichnet und ausgewertet werden.

Eine weitere Erhöhung der Lumineszenzintensität der Mikroorganismen und damit eine Verbesserung der Nachweisempfindlichkeit für die Detektion kann durch Kühlung der Mikroorganismen oder durch Lichteinstrahlung auf die Mikroorganismen erreicht werden.

Gegenstand der Erfindung ist weiterhin ein Kit zum Nachweis biologisch aktiver Substanzen auf einem Test-Träger, insbesondere eine Dünnschichtchromatographie-, Elektrophoreseplatte oder ein Substanzarray, enthaltend Vibrio Fischeri oder eine genetisch modifizierte Variante von V. fischeri in einer für Transport und Lagerung stabilisierten Form, ein oder mehrere Anzuchtmedien oder die Einzelbestandteile für Anzuchtmedien für die Mikroorganismen, dadurch gekennzeichnet, dass das Anzuchtmedium Asparaginsäure in einer Konzentration zwischen 100 mg/l und 500 mg/l als Zusatz zur Stimulierung des Wachstums und optional Borverbindungen, N-Acylhomoserinlactone oder deren biochemische Vorläufersubstanzen oder Chinolone als Lumineszenz stimulierende Substanzen, enthält.

Die Mikroorganismen können durch Gefriertrocknung oder durch Einfrieren mit geeigneten Konservierungsmitteln oder durch Adsorption an geeignete poröse Trägermaterialien oder durch Einschluss in gelförmige Materialien stabilisiert sein.

Der Kit kann weiterhin eine Tauchkammer für die homogene Beschichtung eines Trägers mit den Mikroorganismen enthalten. Bevorzugt weist eine solche Tauchkammer einen Kühlmantel oder Wärmeaustauscher zur Kühlung der Mikroorganismen-Suspension auf.

Die Anwendung einer Tauchkammer für den Beschichtungsschritt erhöht die Lumineszenz der Bakterien-Oberfläche und führt zu gleichmäßiger Beschichtung. Der aus der Beschichtungsqualität folgende gleichmäßige Hintergrund erlaubt eine sichere Erkennung von Substanzzonen.

Bei Anwendung des erfindungsgemäßen Verfahrens innerhalb eines Qualitätssicherungssystems werden in der Regel Aussagen zur Kalibrierung, Validierung und Prüfinittelüberwachung gefordert. Für diese Aufgaben wurde als Bestandteil des erfindungsgemäßen Kits ein Test-Träger konzipiert, der in einem Arbeitsschritt die geforderten Aussagen liefert. Der Test-Träger weist definierte Mengen einer Referenzsubstanz mit bekannter biologischer Wirkung auf. Durch einfaches Tauchen des Test-Trägers in die zu prüfende Suspension lumineszenter Mikroorganismen und nachfolgende Messung mit einem Imagingsystem kann die Qualitätsprüfung bzw. Kalibrierung erfolgen.

In einer bevorzugten Ausführungsform besteht der Test-Träger aus einem Streifen aus Papier, Glas, einem polymeren Material oder Metall mit definiert aufgebrachten Spots der Referenzsubstanzen. Besonders bevorzugt ist der Test-Träger mit porösen Materialien wie Kieselgel beschichtet.

Wird als Referenzsubstanz z.B. 4-Nitrophenol oder ein Kupfersalz verwendet, so werden als Prüfergebnis im Imagingsystem dunkle Zonen an den Positionen der Referenzsubstanzspots ausgewiesen. Damit können sowohl die Funktion des mikrobiologischen Kits wie auch des für die eigentliche Messung notwendigen bildgebenden Systems validiert werden.

Der erfindungsgemäße Kit enthält als Option für Laboratorien oder sonstige Testumgebungen ohne Mikrobiologie-Ausstattung eine Vorrichtung zur vereinfachten Anzucht von Mikroorganismen. In einer bevorzugten Ausführungsform besteht diese aus einem Glas- oder Kunststoffbehälter, das durch seine Formgebung einen guten Sauerstoffaustausch mit der Anzuchtsuspension gewährleistet und einen Magnetrührstab enthält und optional bereits mit einer Anzuchtlösung und/oder einem Verdünnungsmedium und/oder einem Rekonstitutionsmedium gefüllt ist. Nach Animpfen mit Mikroorganismen wird dieser Behälter bei Raumtemperatur auf einen auf geringe Rührgeschwindigkeit eingestellten Magnetrührer gestellt. Auf diese Weise können auf Grundlage des erfindungsgemäßen Verfahrens mit üblichen Laborgeräten Übernachtkulturen hergestellt werden, ohne dass zusätzliche Apparaturen wie z.B. thermostatisierte Schütteltische für die Anzucht erforderlich werden.

In dem erfindungsgemäßen Kit kann das Anzuchtmedium Substanzen, Substanzgemische oder deren Lösungen oder deren biochemische Vorläufer als Zusatz enthalten, die das Wachstums- und/oder die Lumineszenz der Mikroorganismen stimulieren.

In einer Ausfilhrungsfonn enthalten die Anzuchtmedien Dipeptide, Oligopeptide, und optional Borverbindungen, Chinolone oder N-Acylhomoserinlactone oder deren biochemische Vorläufer. In einer bevorzugten Ausführungsformen werden zur Stimulation der Biolumineszenz von *Vibrio fischeri* N-(3-Oxohexanoyl)-L-Homoserinlacton und/oder Borverbindungen eingesetzt.

*Vibrio fischeri* werden in einem Medium angezüchtet, das zwischen 100 mg/l und 500 mg/l Asparaginsäure enthält. Mit solchen Anzuchtmedien können in Übernachtkulturen sehr hohe Zelldichten erreicht werden.

Die Anzuchtmedien können auch Additive enthalten, die die Biolumineszenz regulieren und verlängern. Solche Additive können Oligomere oder Polymere sein, oder aber auch Acrylate, Polyvinylpyridine, Polyethylenglykole, Polyetherderivate, Polysaccharide, Dextrane, modifizierte Zellulosen, Peptide und Proteine.

Alternativ oder gleichzeitig können ein im Kit enthaltenes Verdünnungsmedium zur Verdünnung der Mikroorganismen-Suspension oder Rekonstitutionsmedium zur Herstellung gebrauchsfertiger Mikroorganismen-Suspensionen aus Zellkonzentraten oder stabilisierten Präparationen von Mikroorganismen Wachstums- und/oder Lumineszenz-stimulierende Zusätze oder deren biochemische Vorstufen enthalten wie Borverbindungen, N-Acylhomoserinlactone oder deren biochemische Vorläufersubstanzen oder Chinolone.

Das Verdünnungs- und/oder Rekonstitutionsmedium kann auch Additive enthalten, die die Lumineszenz regulieren und verlängern wie Oligomere oder Polymere, Acrylate, Polyvinylpyridine, Polyethylenglykole, Polyetherderivate, Polysaccharide, Dextrane, modifizierte Zellulosen, Peptide und Proteine.

In einer bevorzugten Ausführungsformen enthalten das Verdünnungs- und/oder Rekonstitutionsmedium zur Stimulation der Lumineszenz von *Vibrio fischeri* N-(3-Oxohexanoyl)-L-Homoserinlacton und/oder Borverbindungen.

Der erfindungsgemäße Kit kann weiterhin eine Lichtquelle und/oder ein Mittel zur Kühlung der Mikroorganismen-Suspension enthalten. Es kann außerdem eine Vorrichtung zur Auftrennung von Proben mittels planarer Separationstechniken wie z.B. Dünnschichtchromatographie oder Gelelektrophorese enthalten.

Dem Kit können außerdem Arbeitsvorschriften zu den einzelnen Arbeitsschritten bei der Präparation der Mikroorganismensuspension und der Lumineszenz-Messung und/oder Informationen zu Anwendungsbeispielen beigelegt sein

Durch das erfindungsgemäße Verfahren und den Kit wird auch bei geringer Konzentration an Mikroorganismen eine hohe Sensitivität für die Detektion möglich und insgesamt eine hohe Wirtschaftlichkeit des Verfahrens erreicht.

Das erfindungsgemäße Verfahren erreicht durch spezielle Anzuchtverfahren, durch gezielte Stimulation der Biolumineszenz und durch die eventuelle Anreicherung im Tauchschritt eine besonders hohe Leistung und erlaubt wegen seiner Robustheit eine besonders einfache Ausführungsform.

Der erfindungsgemäße Kit stellt einen signifikanten Fortschritt gegenüber dem Stand der Technik dar, da er die praktische Anwendung des erfindungsgemäßen Verfahrens für Untersuchungslaboratorien ermöglicht, ohne dort mikrobiologisches Know How und apparative Ausstattung für mikrobiologische Arbeiten vorauszusetzen. Durch die gezielte Steigerung der Biolumineszenz wird eine hohe Robustheit der Messung erreicht. Darüber hinaus kann auf die aufwändige Anreicherung von lumineszenten Mikroorganismen, z.B. durch Zentrifugation, verzichtet werden. Die hohe Lumineszenzleistung lässt sogar eine weitgehende Verdünnung des Detektionsmediums zu.

Zur Steigerung der Lumineszenz beim Verdünnen von Übernachtkulturen oder beim Rekonstituieren von Bakterien, die z.B. durch Gefriertrocknung oder Einfrieren in eine lagerfähige Form gebracht wurden, enthält der Kit Medien mit geeigneten Zusätzen oder Rezepturen für derartige Medien.

Darüber hinaus ermöglicht eine Tauchkammer eine schnelle Beschichtung und erlaubt damit die Untersuchung von sehr rasch einsetzenden biologischen Wirkungen. Gleichzeit bleibt bei dieser schnellen Detektion die Auftrennung der Substanzzonen z.B. auf einer Dünnschichtplatte weitgehend erhalten, da hier nur kurze Diffusionszeiten möglich sind. Ein weiterer Vorteil der Tauchkammer liegt bei der Arbeitshygiene, da hier im Gegensatz zu Spraytechniken keine Aerosole von Mikroorganismen entstehen.

### Abbildungen und Beispiele

Die Figuren zeigen:
- Fig. 1: Lumineszenzbilder einer DC-Platte 1 min und 10 min nach Inkubation mit einer Bakterien-Suspension.
- Fig. 2: Lumineszenzdetektion mit einer Bakterien-Suspension, zum Vergleich hergestellt ohne und mit Zusatz von Asparaginsäure zum Anzuchtmedium.

### Beispiel 1

In Beispiel 1 wird nach dem erfindungsgemäßen Verfahren der spezifische Nachweis von Substanzen, die die Lumineszenz von Mikroorganismen ändern, mit dem erfindungsgemäßen Kit gezeigt.

Zur Detektion der Lumineszenzänderung wurde eine Dünnschichtchromatographie-(DC-) platte, auf der eine Gemischtrennung mittels AMD-Technik [Lit.: K. Burger, *Chemistry of Plant Protection,* **12,** 181-195 (1995)] ausgeführt worden war, mit *Vibrio fischeri* durch Eintauchen des DC-Trägers in eine *Vibrio fischeri*-Suspension beschichtet.

Untersucht wurden Reaktionsproduktgemische, die als bakterientoxische Komponente 4-tert. Butylphenol enthielten sowie Fraktionen aus einem Aufarbeitungsprozess, in dem 4-tert. Butylphenol abgereichert werden sollte. Das folgende Auftrageschema ordnet diese Proben zu:

| | |
|---|---|
| 1 : | 30 ng 4-tert.-Butylphenol Referenz |
| 2 : | 1 µl Reaktionsprodukt 1 |
| 3 : | 1 µl Reaktionsprodukt 2 |
| 4 : | 100 µl Aufarbeitung A von Reaktionsprodukt 1 |
| 5 : | 100 µl Aufarbeitung B von Reaktionsprodukt 1 |
| 6 : | 100 µl Aufarbeitung C von Reaktionsprodukt 1 |
| 7 : | 100 µl Aufarbeitung D von Reaktionsprodukt 1 |
| 8 : | 30 ng 4-tert.-Butylphenol Referenz |

### Herstellung der Vibrio fischeri Tauchsuspension:

Aus einer Stammkonserve *Vibrio fischeri* wurden die Bakterien in einen Erlenmeyerkolben, der 400 ml Anzucht-Medium enthält überimpft. Anschließend wird die beimpfte Anzucht-Lösung in einem Schüttelinkubator bei 28°C über Nacht inkubiert.

Das Anzucht-Medium enthielt:

| | |
|---|---|
| 30 g/l | NaCl |
| 6,1 g/l | NaH₂PO₄ · H₂O |
| 2.75 g/l | K₂HPO₄ · 3 H₂O |
| 0.204 g/l | MgSO₄ · 7 H₂O |
| 0.5 g/l | (NH4)₂HPO₄ |
| 5 g/l | Pepton aus Casein (Merck) |
| 0.5 g/l | Hefe-Extrakt (DIFCO) |
| 3 ml/l | Glyzerin |

und wurde mit Hilfe von 1 N HCl bzw. 1 N NaOH auf pH 7,2 ± 0,2 eingestellt. Das Medium wurde vor Gebrauch bei 121°C für 20 min autoklaviert.

### Lumineszenzdetektion:

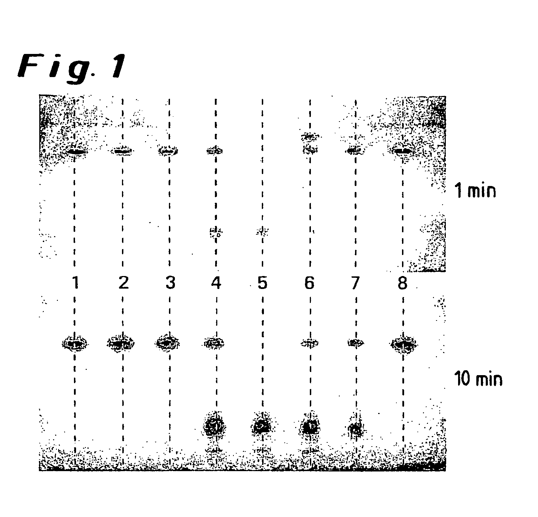

Die Bakteriensuspension wurde in eine CAMAG Tauchkammer für die Dünnschichtchromatographie gegeben. Zur Beschichtung der Dünnschichtplatte wurde diese für wenige Sekunden in die Bakteriensuspension getaucht. Von der beschichteten Platte wurden im feuchten Zustand mit dem Kamerasystem Nightowl (EG&G Berthold) Lumineszenzbilder aufgenommen. Die Auswertung des Fluoreszenzbildes erfolgte visuell am Bildschirm des Videoimagingsystems (Fig. 1). Zur Dokumentation wurden TIFF-Files mit geeigneten Grafikprogrammen formatiert und beschriftet (Adobe Photoshop, MS Powerpoint).

### Ergebnis:

Das Ergebnis der Luminezenzdetektion nach 1 min und 10 min Inkubationszeit ist in Fig. 1 zu sehen. Die Bakterien-toxischen Substanzen werden in der Biolumineszenzdetektion durch Löschung der Biolumineszenz angezeigt. Auf den DC-Bahnen 1 und 8 waren jeweils 30 ng 4-tert. Butylphenol aufgetragen. Dies entspricht etwa dem 4-tert. Butylphenol-Anteil in den Reaktionsprodukten 1 und 2 (Bahnen 2 und 3). Die Aufarbeitungen A bis D (Bahnen 4 - 7), die in der 100fachen Menge aufgetragen wurden, wiesen einen deutlich verringerten Anteil von 4-tert. Butylphenol auf. Bei der hohen Auftragemenge wurden weitere Spurenanteile Lumineszenz-löschender Substanzen sichtbar. Der Vergleich der Lumineszenzbilder nach 1 min und nach 10 min Inkubation auf der DC-Platte ergab für Bahn 6 eine zusätzliche aktive Komponente, die nach längerer Inkubationszeit nicht mehr sichtbar war. Offenbar lag hier eine Substanz vor, die nur kurzzeitig die Vitalität der Bakterienzellen beeinträchtigt. Die schnelle Lumineszenzdetektion nach dem erfindungsgemäßen Verfahren liefert damit Hinweise zu unterschiedlichen Wirkungsweisen von Gemischkomponenten und erhält darüber hinaus die anfängliche Trennschärfe der dünnschichtchromatographischen Trennung. Die diffusionsbedingte Zonenverbreiterung wird bereits beim Vergleich der beiden 9 min auseinanderliegenden Lumineszenzbilder deutlich.

### Beispiel 2

In Beispiel 2 wird nach dem erfmdungsgemäßen Verfahren die Lumineszenzsteigerung infolge verbesserten Wachstums unter Zusatz von Asparaginsäure zum Anzuchtmedium gezeigt.

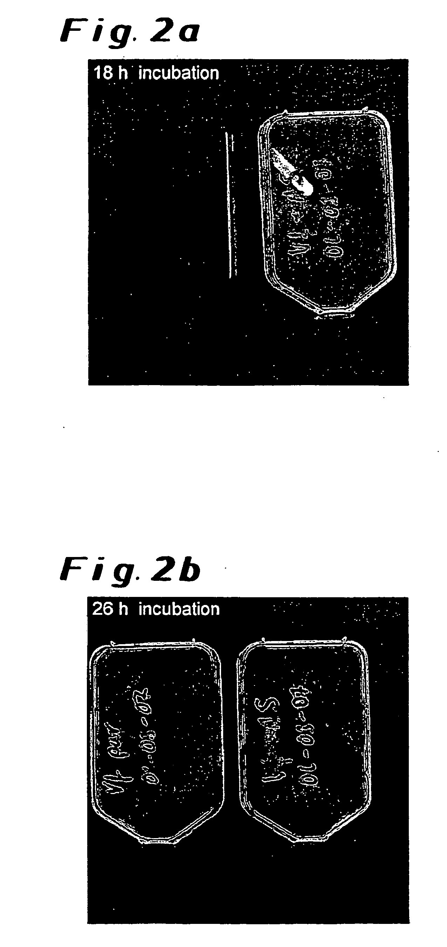

In zwei parallel ausgeführten Anzuchtversuchen wurden Bakteriensuspensionen nach dem unten beschriebenen Verfahren hergestellt. Zur Prüfung des Einflusses von Asparaginsäure war dem Anzuchtmedium in einem der beiden Ansätze 12,5 mg Asparaginsäure zugesetzt worden. Der Effekt des Asparaginsäurezusatzes wurde durch Aufnahme von Lumineszenzbildem mit dem Kamerasystem Nightowl von EG&G Berthold nachgewiesen und ist in den Figuren 2a und 2b dargestellt. Im vorliegenden Fall zeigte der mit Asparaginsäure versetzte Ansatz bereits nach 18 h Inkubation deutliche Lumineszenz (rechtes Anzuchtgefäß in Fig. 2a), während im Asparaginsäure-freien Medium erst zu einem späteren Zeitpunkt die Biolumineszenz eintritt (Fig. 2b).

### Anzuchtverfahren für Vibrio fischeri:

Aus einer Stammkonserve *Vibrio fischeri* wurden die Bakterien in eine Gewebekulturflasche (z.B. Falcon Art. Nr. 353110), die 125 ml Anzucht-Medium und einen Magnetrührstab enthält überimpft. Anschließend wurde die beimpfte Anzucht-Lösung unter langsamem Rühren auf einem Magnetrührer bei Raumtemperatur inkubiert.

## Patentansprüche

1. Verfahren zum Nachweis biologisch aktiver Substanzen enthaltend die Schritte
a) Bereitstellung eines Test-Trägers mit zu prüfenden Substanzen, insbesondere einer Dünnschichtchromatographie-, Elektrophoreseplatte oder eines Substanzarrays,
b) Bereitstellung einer Suspension mit Mikroorganismen der Species Vibrio fischeri oder einer genetisch modifizierten Variante von V. fischeri in einer Anzuchtlösung,
c) Beschichtung des Test-Trägers mit der Mikroorganismen-Suspension durch Tauchen in die Mikroorganismen-Suspension,
d) Nachweis der biologisch aktiven Substanzen auf dem Test-Träger durch Detektion der Lumineszenzänderung der Mikroorganismen-Suspension,
e) Stimulation der Lumineszenz der Mikroorganismen vor oder während der Detektion, **dadurch gekennzeichnet, dass** die Suspension einen Zusatz von Asparaginsäure in einer Konzentration zwischen 100 mg/l und 500 mg/l enthält, sowie dass
f) die Lumineszenz der Mikroorganismen durch Borverbindungen, N-Acylhomoserinlactone oder deren biochemische Vorläufersubstanzen oder Chinolone stimuliert wird.

2. Verfahren nach Anspruch 1, **dadurch gekennzeichnet, dass** die Anzuchtlösung für die Vibrio Fischeri-Suspension die Lumineszenz der Mikroorganismen stimulierende Borverbindungen, N-Acylhomoserinlactone oder deren biochemische Vorläufersubstanzen oder Chinolone enthält.

3. Verfahren nach Anspruch 1 oder 2, **dadurch gekennzeichnet, dass** die Vibrio Fischeri-Suspension aus gefriergetrockneten Mikroorganismen oder gefrorenen Zellkonzentraten durch Rekonstitution mit einem Rekonstitutionsmedium gewonnen wird.

4. Verfahren nach einem der Ansprüche 1 bis 3, **dadurch gekennzeichnet, dass** die Vibrio Fischeri Suspension durch Verdünnen einer Anzuchtlösung für die Mikroorganismen durch ein Verdünnungsmedium erhalten wird.

5. Verfahren nach Anspruch 3 oder 4, **dadurch gekennzeichnet, dass** das Verdünnungsmedium oder das Rekonstitutionsmedium die Lumineszenz der Mikroorganismen gezielt induzierende Borverbindungen, N-Acylhomoserinlactone oder deren biochemische Vorläufersubstanzen oder Chinolone enthält.

6. Verfahren nach Anspruch 1 bis 5, **dadurch gekennzeichnet, dass** eine Lumineszenzsteigerung weiterhin durch eine hohe Zelldichte von mehr als 2*10⁹ Zellen/ml in der Vibrio Fischeri-Suspension erreicht wird.

7. Verfahren nach Anspruch 1 bis 6, **dadurch gekennzeichnet, dass** die Beschichtung des Trägers homogen erfolgt.

8. Verfahren nach Anspruch 7, **dadurch gekennzeichnet, dass** zur Beschichtung des Trägers eine Tauchsuspension eingesetzt wird, wobei es sich dabei um eine bis auf das mehr als fünffache Volumen verdünnten Suspension einer Übernachtkultur von Vibrio fischeri handelt.

9. Verfahren nach Anspruch 1 bis 8, **dadurch gekennzeichnet, dass** die Detektion der Lumineszenz der Vibrio Fischeri-Suspension durch photographische Verfahren oder Imagingtechniken erfolgt.

10. Verfahren nach Anspruch 1 bis 9, **dadurch gekennzeichnet, dass** die biologisch aktiven Substanzen eine stimulierende oder inhibierende Wirkung auf die Lumineszenz der Vibrio Fischeri-Suspension haben.

11. Verfahren nach Anspruch 10, **dadurch gekennzeichnet, dass** der zeitliche Verlauf der Inhibierung bzw. Stimulierung der Lumineszenz der Vibrio Fischeri-Suspension aufgezeichnet wird und ausgewertet wird.

12. Verfahren nach Anspruch 1 bis 11, **dadurch gekennzeichnet, dass** die Vibrio Fischeri-Suspension vor und/oder während des Detektionsvorgangs einer Kühlung unterworfen werden.

13. Verfahren nach Anspruch 1 bis 12, **dadurch gekennzeichnet, dass** die Vibrio Fischeri-Suspension vor und/oder während des Detektionsvorgangs einer Lichteinstrahlung unterworfen werden.

14. Kit zum Nachweis biologisch aktiver Substanzen auf einem Test-Träger, insbesondere eine Dünnschichtchromatographie-, Elektrophoreseplatte oder ein Substanzarray, enthaltend Vibrio Fischeri oder eine genetisch modifizierte Variante von V. fischeri in einer für Transport und Lagerung stabilisierten Form, ein oder mehrere Anzuchtmedien oder die Einzelbestandteile für Anzuchtmedien für die Mikroorganismen, **dadurch gekennzeichnet, dass** das Anzuchtmedium Asparaginsäure in einer Konzentration zwischen 100 mg/l und 500 mg/l als Zusatz zur Stimulierung des Wachstums und optional Borverbindungen, N-Acylhomoserinlactone oder deren biochemische Vorläufersubstanzen oder Chinolone als Lumineszenz stimulierende Substanzen, enthält.

15. Kit nach Anspruch 14, **dadurch gekennzeichnet, dass** die Mikroorganismen durch Gefriertrocknung oder durch Einfrieren mit geeigneten Konservierungsmitteln oder durch Adsorption an geeignete poröse Trägermaterialien oder durch Einschluss in gelförmige Materialien stabilisiert wurden.

16. Kit nach Anspruch 14 oder 15, **dadurch gekennzeichnet, dass** er eine Tauchkammer für die homogene Beschichtung eines Trägers mit den Mikroorganismen enthält.

17. Kit nach Anspruch 16, **dadurch gekennzeichnet, dass** die Tauchkammer einen Kühlmantel oder Wärmeaustauscher zur Kühlung der Mikroorganismen-Suspension aufweist.

18. Kit nach Anspruch 14 bis 17, **dadurch gekennzeichnet, dass** er einen Test-Träger, auf dem definierte Referenzsubstanzmengen mit bekannter biologischer Wirkung aufgebracht sind, enthält.

19. Kit nach Anspruch 14 bis 18, **dadurch gekennzeichnet, dass** der Test-Träger als Referenzsubstanz 4-Nitrophenol oder ein Kupfersalz enthält.

20. Kit nach Anspruch 14 bis 19, **dadurch gekennzeichnet, dass** er weiterhin eine Vorrichtung für die Anzucht von Mikroorganismen enthaltend einen Glas- oder Kunststoffbehälter, enthält.

21. Kit nach Anspruch 20, **dadurch gekennzeichnet, dass** der Glas- oder Kunststoffbehälter durch seine Formgebung einen guten Sauerstoffaustausch mit der Anzuchtsuspension gewährleistet und einen Magnetrührstab aufweist.

22. Kit nach Anspruch 20 oder 21, **dadurch gekennzeichnet, dass** die Behälter eine Anzuchtlösung und/oder ein Verdünnungsmedium und/oder ein Rekonstitutionsmedium enthalten.

23. Kit nach Anspruch 14 bis 22, **dadurch gekennzeichnet, dass** die Anzuchtmedien die Lumineszenz der Mikroorganismen gezielt induzierende Borverbindungen, N-Acylhomoserinlactone oder deren biochemische Vorläufersubstanzen oder Chinolone enthalten.

24. Kit nach Anspruch 14 bis 23, **dadurch gekennzeichnet, dass** er Verdünnungsmedien zur Verdünnung von Mikroorganismen-Suspensionen enthält.

25. Kit nach Anspruch 24, **dadurch gekennzeichnet, dass** die Verdünnungsmedien Asparaginsäure in einer Konzentration zwischen 100 mg/l und 500 mg/l als Zusatz zur Stimulierung des Wachstums und optional Borverbindungen, N-Acylhomoserinlactone oder deren biochemische Vorläufersubstanzen oder Chinolone als Lumineszenz stimulierende Substanzen, enthalten.

26. Kit nach Anspruch 14 bis 24, **dadurch gekennzeichnet, dass** er Rekonstitutionsmedien zur Herstellung gebrauchsfertiger Mikroorganismen-Suspensionen aus Zellkonzentraten oder stabilisierten Präparationen von Mikroorganismen enthält.

27. Kit nach Anspruch 26, **dadurch gekennzeichnet, dass** die Rekonstitutionsmedien Asparaginsäure in einer Konzentration zwischen 100 mg/l und 500 mg/l als Zusatz zur Stimulierung des Wachstums und optional Borverbindungen, N-Acylhomoserinlactone oder deren biochemische Vorläufersubstanzen oder Chinolone als Lumineszenz stimulierende Substanzen, enthalten.

28. Kit nach Anspruch 14 bis 27, **dadurch gekennzeichnet, dass** er weiterhin eine Lichtquelle enthält.

29. Kit nach Anspruch 14 bis 28, **dadurch gekennzeichnet, dass** er weiterhin ein Kühlelement oder einen Wärmeaustauscher für die Kühlung der Mikroorganismen-Suspension enthält.

30. Kit nach Anspruch 14 bis 29, **dadurch gekennzeichnet, dass** er weiterhin eine Vorrichtung zur Auftrennung von Proben mit planaren Separationstechniken wie der Dünnschichtchromatographie oder der Gelelektrophorese enthält.

## Claims

1. Method for detecting biologically active substances comprising the steps of
a) providing a test support carrying substances to be tested, in particular a thin-layer chromatography plate, electrophoresis plate or a substance array,
b) providing a suspension containing microorganisms of the species Vibrio fischeri or a genetically modified variant of V. fischeri in a growth solution,
c) coating the test support with the microorganism suspension by dipping into the microorganism suspension,
d) detecting the biologically active substances on the test support by detecting change in luminescence of the microorganism suspension,
e) stimulating the luminescence of the microorganisms before or during detection, **characterized in that** the suspension contains an addition of aspartic acid in a concentration between 100 mg/l and 500 mg/l, and also **in that**
f) the luminescence of the microorganisms is stimulated by boron compounds, N-acylhomoserine lactones or biochemical precursor substances thereof or quinolones.

2. Method according to Claim 1, **characterized in that** the growth solution for the Vibrio fischeri suspension contains boron compounds, N-acylhomoserine lactones or biochemical precursor substances thereof or quinolones stimulating the luminescence of the microorganisms.

3. Method according to Claim 1 or 2, **characterized in that** the Vibrio fischeri suspension is obtained from freeze-dried microorganisms or frozen cell concentrates by reconstitution using a reconstitution medium.

4. Method according to one of Claims 1 to 3, **characterized in that** the Vibrio fischeri suspension is obtained by diluting a growth solution for the microorganisms by a dilution medium.

5. Method according to Claim 3 or 4, **characterized in that** the dilution medium or the reconstitution medium contains boron compounds, N-acylhomoserine lactones or biochemical precursor substances thereof or quinolones specifically inducing the luminescence of the microorganisms.

6. Method according to Claim 1 to 5, **characterized in that** a luminescence increase is further achieved by a high cell density of greater than 2 × 10⁹ cells/ml in the Vibrio fischeri suspension.

7. Method according to Claim 1 to 6, **characterized in that** the support is coated homogeneously.

8. Method according to Claim 7, **characterized in that**, for coating the support, use is made of a dipping suspension, this being an up to more than five-fold dilution by volume of a suspension of an overnight culture of Vibrio fischeri.

9. Method according to Claim 1 to 8, **characterized in that** the luminescence of the Vibrio fischeri suspension is detected by photographic methods or imaging techniques.

10. Method according to Claim 1 to 9, **characterized in that** the biologically active substances have a stimulating or inhibiting effect on the luminescence of the Vibrio fischeri suspension.

11. Method according to Claim 10, **characterized in that** the time course of the inhibition or stimulating of the luminescence of the Vibrio fischeri suspension is recorded and analysed.

12. Method according to Claim 1 to 11, **characterized in that** the Vibrio fischeri suspension is subjected to cooling before and/or during the detection process.

13. Method according to Claim 1 to 12, **characterized in that** the Vibrio fischeri suspension is subjected to light irradiation before and/or during the detection process.

14. Kit for detecting biologically active substances on a test support, in particular a thin-layer chromatography plate, an electrophoresis plate, or a substance array containing Vibrio fischeri or a genetically modified variant of V. fischeri in a form stabilized for transport and storage, one or more growth media or the individual constituents for growth media for the microorganisms, **characterized in that** the growth medium contains aspartic acid in a concentration between 100 mg/l and 500 mg/l as addition for stimulating growth, and optionally boron compounds, N-acylhomoserine lactones or biochemical precursor substances thereof or quinolones as luminescence-stimulating substances.

15. Kit according to Claim 14, **characterized in that** the microorganisms were stabilized by freeze drying or by freezing together with suitable preservatives or by adsorption to suitable porous support materials or by inclusion in gel-like materials.

16. Kit according to Claim 14 or 15; **characterized in that** it contains a dipping chamber for the homogeneous coating of a support by the microorganisms.

17. Kit according to Claim 16, **characterized in that** the dipping chamber has a cooling jacket or heat exchanger for cooling the microorganism suspension.

18. Kit according to Claim 14 to 17, **characterized in that** it contains a test support to which defined amounts of reference substances having a known biological action have been applied.

19. Kit according to Claim 14 to 18, **characterized in that** the test support contains 4-nitrophenol or a copper salt as reference substance.

20. Kit according to Claim 14 to 19, **characterized in that** it further contains a device for the growth of microorganisms which contains a glass or plastic container.

21. Kit according to Claim 20, **characterized in that** the glass or plastic container, as a result of its design, ensures good oxygen exchange with the growth suspension and has a magnetic stirring bar.

22. Kit according to Claim 20 or 21, **characterized in that** the containers contain a growth solution and/or a dilution medium and/or a reconstitution medium.

23. Kit according to Claim 14 to 22, **characterized in that** the growth media contain boron compounds, N-acylhomoserine lactones or biochemical precursor substances thereof or quinolones which specifically induce the luminescence of the microorganisms.

24. Kit according to Claim 14 to 23, **characterized in that** it contains dilution media for diluting microorganism suspensions.

25. Kit according to Claim 24, **characterized in that** the dilution media contain aspartic acid in a concentration between 100 mg/l and 500 mg/l as addition for stimulating growth, and optionally boron compounds, N-acylhomoserine lactones or biochemical precursor substances thereof or quinolones as luminescence-stimulating substances.

26. Kit according to Claim 14 to 24, **characterized in that** it contains reconstitution media for producing ready-to-use microorganism suspensions from cell concentrates or stabilized preparations of microorganisms.

27. Kit according to Claim 26, **characterized in that** the reconstitution media contain aspartic acid in a concentration between 100 mg/l and 500 mg/l as addition for stimulating growth, and optionally boron compounds, N-acylhomoserine lactones or biochemical precursor substances thereof or quinolones as luminescence-stimulating substances.

28. Kit according to Claim 14 to 27, **characterized in that** it further contains a light source.

29. Kit according to Claim 14 to 28, **characterized in that** it further contains a cooling element or a heat exchanger for cooling the microorganism suspension.

30. Kit according to Claim 14 to 29, **characterized in that** it further contains a device for fractionating samples using planar separation techniques such as thin-layer chromatography or gel electrophoresis.

## Revendications

1. Procédé pour la détection de substances possédant une activité biologique, qui comprend les stades opératoires suivants :
a) préparation d'un support pour essais avec les substances soumises à l'essai, en particulier d'une plaque de chromatographie sur couche mince, d'une plaque d'électrophorèse ou d'une série de substances,
b) préparation d'une suspension contenant des micro-organismes de l'espèce Vibrio fischeri ou d'une variante génétiquement modifiée de V. fischeri dans une solution de culture,
c) revêtement du support pour essais par la suspension de micro-organismes, par immersion de ce support dans la suspension de micro-organismes,
d) détection des substances possédant une activité biologique sur le support pour essais par la variation de luminescence de la suspension de micro-organismes,
e) stimulation de la luminescence des micro-organismes, avant ou durant la détection, ce procédé **se caractérisant en ce que** la suspension est additionnée d'acide aspartique à une concentration allant de 100 mg/l à 500 mg/l, et **en ce que**
f) la luminescence des micro-organismes est stimulée par des dérivés du bore, des lactones de N-acylhomosérines ou leurs produits précurseurs biochimiques ou par des quinolones.

2. Procédé selon la revendication 1, **caractérisé en ce que** la solution de culture pour la suspension de Vibrio fischeri contient des composés du bore, des lactones de N-acylhomosérines ou leurs produits précurseurs biochimiques ou des quinolones qui stimulent la luminescence des micro-organismes.

3. Procédé selon la revendication 1 ou 2, **caractérisé en ce que** la suspension de Vibrio fischeri a été obtenue à partir de micro-organismes lyophilisés ou de concentrés cellulaires congelés par reconstitution dans un milieu approprié.

4. Procédé selon l'une des revendications 1 à 3, **caractérisé en ce que** la suspension de Vibrio fischeri a été obtenue par dilution d'une solution de culture des micro-organismes par un milieu diluant.

5. Procédé selon la revendication 3 ou 4, **caractérisé en ce que** le milieu diluant ou le milieu de reconstitution contient des dérivés du bore, des lactones de N-acylhomosérines ou leurs produits précurseurs biochimiques ou des quinolones qui induisent la luminescence des micro-organismes.

6. Procédé selon les revendications 1 à 5, **caractérisé en ce que** l'on parvient encore à augmenter la luminescence en opérant avec une forte densité cellulaire, supérieure à 2 x 10⁹ cellules/ml dans la suspension de Vibrio fischeri.

7. Procédé selon les revendications 1 à 6, **caractérisé en ce que** le revêtement du support est homogène.

8. Procédé selon la revendication 7, **caractérisé en ce que**, pour le revêtement du support, on utilise pour l'immersion une suspension, diluée d'une fois à plus de cinq fois son volume, par une culture d'une nuit de Vibrio fischeri.

9. Procédé selon les revendications 1 à 8, **caractérisé en ce que** la détection de la luminescence de la suspension de Vibrio fischeri est réalisée par des techniques photographiques ou par des techniques d'imagerie.

10. Procédé selon les revendications 1 à 9, **caractérisé en ce que** les substances possédant l'activité biologique ont un effet stimulant ou inhibiteur sur la luminescence de la suspension de Vibrio fischeri.

11. Procédé selon la revendication 10, **caractérisé en ce que** l'on enregistre et on évalue la variation dans le temps de l'inhibition ou de la stimulation de la luminescence de la suspension de Vibrio fischeri.

12. Procédé selon les revendications 1 à 11, **caractérisé en ce que** la suspension de Vibrio fischeri, avant et/ou durant l'opération de détection, est soumise à un refroidissement.

13. Procédé selon les revendications 1 à 12, **caractérisé en ce que** la suspension de Vibrio fischeri, avant et/ou durant l'opération de détection, est soumise à irradiation par la lumière.

14. Kit pour la détection de substances possédant une activité biologique sur un support pour essais, en particulier une plaque de chromatographie sur couche mince, une plaque d'électrophorèse ou une série de substances, contenant Vibrio fischeri ou une variante génétiquement modifiée de V. fischeri sous une forme stabilisée pour le transport et la conservation, un ou plusieurs milieux de culture ou les constituants individuels de milieux de culture pour les micro-organismes, **caractérisé en ce que** le milieu de culture contient de l'acide aspartique à une concentration de 100 mg/l à 500 mg/l, cet additif servant à stimuler la croissance, ainsi que facultativement des dérivés du bore, des lactones de N-acylhomosérines ou leurs produits précurseurs biochimiques ou des quinolones, en tant que substances stimulant la luminescence.

15. Kit selon la revendication 14, **caractérisé en ce que** les micro-organismes ont été stabilisés par lyophilisation ou par congélation avec des conservateurs appropriés ou par adsorption sur des matières poreuses appropriées ou par inclusion dans des matières gélifiables.

16. Kit selon la revendication 14 ou 15, **caractérisé en ce qu'**il contient une chambre d'immersion pour le revêtement homogène d'un support par les micro-organismes.

17. Kit selon la revendication 16, **caractérisé en ce que** la chambre d'immersion est équipée d'une enveloppe refroidisseuse ou d'un échangeur de chaleur servant à refroidir la suspension de micro-organismes.

18. Kit selon les revendications 14 à 17, **caractérisé en ce qu'**il contient un support pour essais sur lequel on a appliqué des quantités définies de substances témoins possédant une activité biologique connue.

19. Kit selon les revendications 14 à 18, **caractérisé en ce que** le support pour essais contient en tant que substance témoin le 4-nitrophénol ou un sel de cuivre.

20. Kit selon les revendications 14 à 19, **caractérisé en ce qu'**il contient en outre un dispositif pour la culture de micro-organismes contenant lui-même un récipient de verre ou de matières plastiques.

21. Kit selon la revendication 20, **caractérisé en ce que** le récipient de verre ou de matière plastique, par sa forme, assure un bon échange d'oxygène avec la suspension de culture, et contient un barreau d'agitation magnétique.

22. Kit selon la revendication 20 ou 21, **caractérisé en ce que** les récipients contiennent une solution de culture et/ou un milieu diluant et/ou un milieu reconstituant.

23. Kit selon les revendications 14 à 22, **caractérisé en ce que** les milieux de culture contiennent des dérivés du bore, des lactones de N-acylhomosérines ou leurs produits précurseurs biochimiques ou des quinolones qui induisent la luminescence des micro-organismes.

24. Kit selon les revendications 14 à 23, **caractérisé en ce qu'**il contient des milieux diluants servant à diluer la suspension de micro-organismes.

25. Kit selon la revendication 24, **caractérisé en ce que** les milieux diluants contiennent de l'acide aspartique à une concentration de 100 mg/l à 500 mg/l, cet additif servant à stimuler la croissance, et facultativement des dérivés du bore, des lactones de N-acylhomosérine ou leurs produits précurseurs biochimiques ou des quinolones, substances qui stimulent la luminescence.

26. Kit selon les revendications 14 à 24, **caractérisé en ce qu'**il contient des milieux reconstituants pour la préparation de suspensions de micro-organismes prêtes à l'emploi à partir de concentrés cellulaires ou de préparations stabilisées de micro-organismes.

27. Kit selon la revendication 26, **caractérisé en ce que** les milieux reconstituants contiennent de l'acide aspartique à une concentration de 100 mg/l à 500 mg/l, cet additif servant à stimuler la croissance, et facultativement des dérivés du bore, des lactones de N-acylhomosérines ou leurs produits précurseurs biochimiques ou des quinolones, substances qui stimulent la luminescence.

28. Kit selon les revendications 14 à 27, **caractérisé en ce qu'**il contient en outre une source de lumière.

29. Kit selon les revendications 14 à 28, **caractérisé en ce qu'**il contient en outre un élément réfrigérant ou un échangeur de chaleur servant à refroidir la suspension de micro-organismes.

30. Kit selon les revendications 14 à 29, **caractérisé en ce qu'**il contient en outre un dispositif pour la séparation d'échantillons par des techniques de séparation planaires telles que la chromatographie sur couche mince ou l'électrophorèse sur gel.
